# FASCICULE DE BREVET EUROPEEN

(11) **EP 0 755 696 B1**
(45) Date de publication et mention de la délivrance du brevet: **18.02.2004**
(21) Numéro de dépôt: 96401612.5
(22) Date de dépôt: 19.07.1996
(51) Int. Cl.: A61N 1/368, A61N 1/362

(54) **Stimulateur cardiaque double chambre**
Zweikammer-Herzschrittmacher
Dual chamber pacemaker

(30) Priorité: 25.07.1995 FR 9509031
(43) Date de publication de la demande: 29.01.1997
(73) Titulaire: ELA MEDICAL (Société anonyme), 92541 Montrouge (FR)
(72) Inventeur: Limousin, Marcel, 92120 Montrouge (FR); Bonnet, Jean-Luc, 92170 Vanves (FR)
(74) Mandataire: Dupuis-Latour, Dominique

(56) Documents cités:
- EP-A- 0 318 304
- EP-A- 0 488 840
- EP-A- 0 661 076
- WO-A-92/03184
- WO-A-93/14815
- US-A- 5 403 356

## Description

La présente invention concerne le traitement des Extra-Systoles Auriculaires (ESA) dans un stimulateur cardiaque, ainsi que le fonctionnement corrélatif en mode de repli et lors de la resynchronisation à l'issue d'une phase de repli.

Ces questions ont notamment été abordées par le EP-A-0 488 840 au nom de ELA Médical, qui décrit un mode de traitement des ESA et un fonctionnement en repli, correspondant à ce qui est mis en oeuvre dans le stimulateur *Chorus II 6234* de cette même société.

Essentiellement, ce procédé connu des traitements des ESA, destiné à un stimulateur cardiaque double chambre comportant un mode de désynchronisation de la stimulation ventriculaire lorsque le rythme auriculaire dépasse un niveau admissible, puis de resynchronisation progressive en cas de retour du rythme auriculaire au niveau admissible, comporte les étapes suivantes :
(a) à chaque détection d'une dépolarisation auriculaire, on définit une fenêtre de Détection de l'Accélération du Rythme Auriculaire ou DARA dont la durée est fonction du rythme auriculaire antérieur, évalué d'après la durée d'un ou plusieurs des intervalles auriculaires précédents, et
(b) à la détection de la dépolarisation auriculaire suivante, on compte l'écoulement d'un Intervalle d'Échappement Auriculaire ou IEA et
(c) si cette dépolarisation est présente dans la fenêtre de DARA, on commande si nécessaire une stimulation auriculaire consécutive en fonction de l'IEA ainsi défini,

Ce mode opératoire n'est cependant pas dépourvu d'inconvénients.

En premier lieu, les ESA pouvant conduire à des Troubles du Rythme Auriculaire (TdRA), il est important de les reconnaître avec précision et de bien distinguer entre une véritable ESA et une simple accélération physiologique du rythme cardiaque. À cet égard, avec le procédé connu le traitement des ESA, on constate en particulier que :
- une DARA fixée à 25 % ne permet pas de discriminer entre une accélération physiologique (début d'effort) et une ESA isolée, pouvant ainsi entraîner une mauvaise réponse de l'appareil ;
- d'autre part, sur une ESA isolée, le Délai Atrio-Ventriculaire (DAV) de 31 ms qui suit cette ESA est mal supporté par le patient.

En second lieu, en cas de TdRA, pour éviter que le trouble du rythme ne s'installe, il est nécessaire de modifier le comportement du stimulateur suite à la détection d'une ESA. Mais :
― à détection d'une Fibrillation Auriculaire (FA), l'appareil commute son mode de fonctionnement de DDD en VVI (mode de repli); en cas de perte ultérieure de la détection, il repasse en mode DDD et l'analyse qu'il poursuit ne lui permet pas de repasser en VVI;
― enfin, à l'issue du mode de repli, c'est-à-dire quand la fréquence auriculaire est passée au-dessous de la fréquence maximale de synchronisation, il y a resynchronisation, mais le délai pour le retour en DDD est parfois trop long, ce qui peut favoriser une conduction rétrograde ou une bradycardie, avec risque de retour du TdRA.

La présente invention propose un stimulateur comprenant des moyens de traitement des ESA et de fonctionnement en repli et resynchronisation qui pallie l'ensemble des inconvénients ainsi rencontrés avec les appareils connus.

Le stimulateur de l'invention est du type général exposé plus haut, divulgué par exemple dans le EP-A-0 488 840.

Le perfectionnement apporté par l'invention est exposé dans la partie caractérisante de la revendication 1. Les sous-revendications exposent des modes de mise en oeuvre avantageux.

On va maintenant donner un exemple détaillé de mise en oeuvre de l'invention, en référence à la figure unique annexée qui montre les différentes périodes impliquées dans l'invention en cas d'ESA. Le tracé du haut correspond à une onde P spontanée présente hors DARA, tandis que le tracé du bas correspond à l'absence d'une telle onde P spontanée, entraînant le déclenchement d'une stimulation auriculaire par l'appareil.

Le repli est basé sur la capacité à différencier une activité sinusale normale d'une Extra-Systole Auriculaire (ESA). La fenêtre de Détection de l'Accélération du Rythme Auriculaire (DARA) permet de faire cette différenciation. La fenêtre de DARA est une période qui démarre sur un événement auriculaire et dure 75 % de l'intervalle sinusal précédent s'il correspond à un rythme supérieur ou égal à 80 min⁻¹ ou 62,5 % de l'intervalle sinusal précédent s'il correspond à un rythme inférieure à 80 min⁻¹. Si l'intervalle auriculaire précédent est stimulé ou ralenti de plus de 25 % par rapport au dernier cycle sinusal, la fenêtre de DARA est égale à 75 % ou 62,5 % de la moyenne des huit derniers cycles reconnus comme sinusaux.

Une activité sinusale normale est définie comme un événement auriculaire qui est présent en dehors de la fenêtre de DARA. Une ESA est un événement auriculaire détecté dans la fenêtre de DARA.

Lorsqu'une ESA est détectée, on ne démarre pas de Délai Atrio-Ventriculaire (DAV), on ne stimule pas le ventricule et on recycle l'Intervalle d'Echappement Auriculaire (IEA) d'une valeur égale à la valeur de la fenêtre de DARA. En recyclant l'IEA, on évite une stimulation auriculaire prématurée qui pourrait induire une arythmie auriculaire et on donne le temps à un autre événement auriculaire d'arriver dans les cas d'arythmie auriculaire.

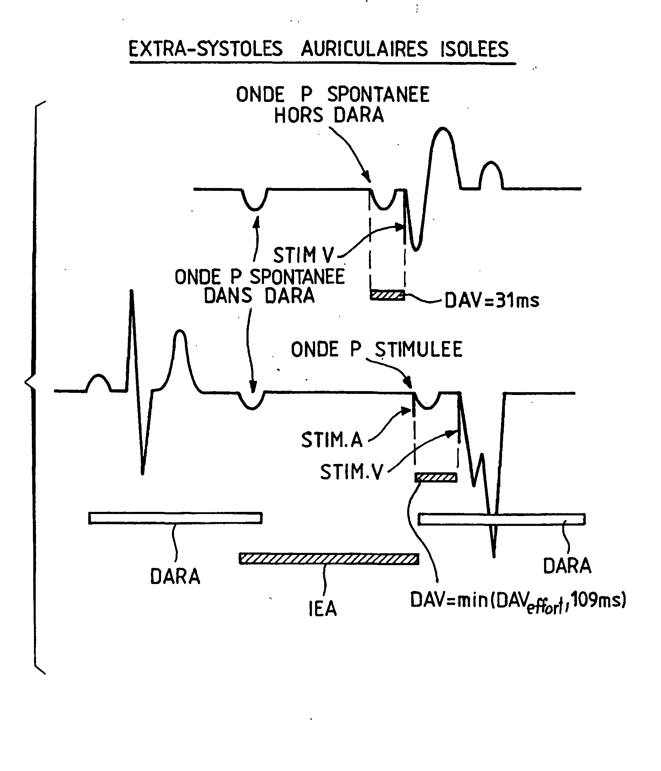

Cas d'une ESA isolée : Après la détection d'une ESA, on recycle l'IEA. Si aucun événement auriculaire n'est détecté avant la fin de l'IEA, l'oreillette est stimulée et le DAV d'effort est appliqué s'il est inférieur ou égal à 109 ms, sinon un DAV de 109 ms est appliqué (voir tracé du bas de la figure 1). L'utilisation d'un DAV assez court minimise l'allongement excessif de l'intervalle d'échappement ventriculaire. La fenêtre de DARA suivante est égale à 75 % ou 62,5 % de la moyenne des huit cycles sinusaux précédents.

Cas d'un Trouble du Rythme Auriculaire (TdRA) : Si une activité auriculaire est détectée après l'ESA et avant la fin de l'IEA, on entre dans une phase de suspicion de TdRA. Un DAV de 31 ms est déclenché sur une détection auriculaire pendant l'IEA (voir tracé du haut de la figure 1). Par contre, sur une stimulation auriculaire à la fin de l'IEA, le DAV d'effort est appliqué s'il est inférieur ou égal à 109 ms, sinon un DAV de 109 ms est appliqué. La fréquence de stimulation ventriculaire est limitée à 120 min⁻¹, ou à la valeur programmée de la fréquence maximale si elle st inférieure à 120 min⁻¹. La valeur de la fenêtre de DARA est celle utilisée avant l'entrée dans la phase de suspicion de TdRA.

Lorsqu'une activité auriculaire est détectée pendant la DARA, on recycle la période de détection auriculaire à 31 ms avant la fin de l'intervalle minimum de stimulation (intervalle correspondant à la limitation de fréquence de stimulation ventriculaire).

Ainsi, en fonction de la fréquence du TdRA, on assure un fonctionnement stable en N:1. La stimulation ventriculaire est limitée à 120 min⁻¹ ou à la fréquence maximale programmée, si cette dernière est plus basse.

Suspicion de TdRA par le capteur : Cette fonction est automatiquement disponible si le repli est programmé à "Oui" et le mode d'asservissement à "RRauto". Elle permet de détecter les troubles du rythme auriculaire qui auraient échappé à la suspicion de TdRA par la fenêtre de DARA.

On considère qu'un cycle cardiaque est en phase de suspicion de TdRA par le capteur si le rythme auriculaire est supérieur ou égal à 120 min⁻¹ alors que la fréquence d'asservissement est inférieure à 98 min⁻¹.

Confirmation d'un TdRA : Tous les 32 cycles cardiaques, on compte le nombre de cycles en phase de suspicion de TdRA et le nombre de cycles en phase de suspicion de TdRA par le capteur. Un TdRA est confirmé si l'une des trois conditions suivantes est vérifiée :
― sur le dernier groupe de 32 cycles, 28 cycles ou plus sont en phase de suspicion de TdRA (cas d'un TdRA avec une bonne détection auriculaire),
― sur les 2 derniers groupes de 32 cycles, 18 cycles par groupe, ou plus, sont en phase de suspicion de TdRA (cas d'un TdRA avec une détection auriculaire moyenne),
― sur les 4 derniers groupes de 32 cycles, 24 cycles par groupe, ou plus, sont en phase de suspicion de TdRA par le capteur (cas d'un TdRA qui démarre lentement et qui n'est pas détecté par la fenêtre de DARA).

Repli : Lorsqu'un trouble du rythme auriculaire est confirmé, on fonctionne en mode VDI. L'intervalle d'échappement ventriculaire est allongé de 31 ms tous les 12 cycles jusqu'à ce que la fréquence de stimulation atteigne la fréquence de base, la fréquence de sommeil ou la fréquence d'asservissement (si le mode d'asservissement est RRauto, RRfigée, DDD/VVIRauto ou DDD/VVIRfigée). Si aucune activité auriculaire n'est détectée, on stimule l'oreillette et le ventricule avec un DAV égal au DAV d'effort s'il est inférieur à 109 ms ; sinon, un DAV de 109 ms est utilisé. Le but est d'assurer une synchronisation auriculo-ventriculaire dès que le trouble du rythme auriculaire s'arrête.

Tous les 12 cycles cardiaques, on mesure la fréquence auriculaire et la fréquence ventriculaire. Tant que l'une de ces deux fréquences est supérieure à 120 min⁻¹ ou à la fréquence maximale programmée (si elle est inférieure à 120 min⁻¹), on reste en repli. Dès que les fréquences auriculaires et ventriculaires deviennent inférieures à 120 min⁻¹ ou à la fréquence maximale programmée (si elle est inférieure à 120 min⁻¹), on raccourcit l'intervalle d'échappement ventriculaire de 63 ms tous les 12 cycles jusqu'à ce qu'il atteigne la fréquence auriculaire (ou la fréquence d'asservissement). Oreillette et ventricule sont alors resynchronisés.

On notera par ailleurs que le mode d'asservissement est DDD/VVIRauto ou DDD/VVIRfigée, le mode de fonctionnement est non asservi tant que le rythme auriculaire est normal. Dès que l'on détecte un trouble du rythme auriculaire, l'asservissement est appliqué pendant la phase de repli. Enfin, si le mode d'asservissement est DD/VVIRauto, la suspicion de TdRA par le capteur n'est pas appliqué.

## Revendications

1. Un stimulateur cardiaque double chambre comportant un mode de désynchronisation de la stimulation ventriculaire lorsque le rythme auriculaire dépasse un niveau admissible, puis de resynchronisation progressive en cas de retour du rythme auriculaire au niveau admissible, ce stimulateur comprenant :
a) des moyens aptes, à chaque détection d'une dépolarisation auriculaire, à définir une fenêtre de Détection de l'Accélération du Rythme Auriculaire ou DARA dont la durée est fonction du rythme auriculaire antérieur, évalué d'après la durée d'un ou plusieurs des intervalles auriculaires précédents, et
b) des moyens aptes, à la détection de la dépolarisation auriculaire suivante, à compter l'écoulement d'un Intervalle d'Échappement Auriculaire ou IEA, et
c) des moyens aptes, si cette dépolarisation est présente dans la fenêtre de DARA, à commander si nécessaire une stimulation auriculaire consécutive en fonction de l'IEA ainsi défini,
stimulateur **caractérisé en ce que** les moyens aptes à définir une fenêtre de DARA comprennent des moyens aptes à adapter la durée de la fenêtre de DARA en fonction du rythme auriculaire antérieur en définissant une fenêtre d'une première durée ou une fenêtre d'une deuxième durée plus courte que ladite première durée, la première durée étant définie pour des premières valeurs de rythme auriculaire antérieur et la deuxième durée étant définie pour des deuxièmes valeurs de rythme auriculaire antérieur plus lentes que lesdites premières valeurs,
de manière à permettre une discrimination entre Extra-Systoles Auriculaires ou ESA et accélérations physiologiques du rythme auriculaire.

2. Le stimulateur de la revendication 1, dans lequel les moyens aptes à définir une fenêtre de DARA comprennent des moyens aptes, pour déterminer si le rythme auriculaire antérieur appartient auxdites premières valeurs de rythme auriculaire antérieur ou auxdites deuxièmes valeurs de rythme auriculaire antérieur, à comparer la valeur du rythme auriculaire antérieur à un seuil et à définir ladite fenêtre d'une première durée lorsque le rythme dépasse le seuil et ladite fenêtre d'une deuxième durée dans le cas contraire.

3. Le stimulateur de la revendication 1, dans lequel la durée de la fenêtre de DARA est égale à une fraction de l'intervalle séparant deux dépolarisations auriculaires physiologiques successives antérieures.

4. Le stimulateur de la revendication 1, dans lequel la durée de la fenêtre de DARA est égale à une fraction de la moyenne d'un nombre prédéterminé des intervalles séparant deux dépolarisations auriculaires physiologiques successives antérieures.

5. Le stimulateur de la revendication 3 ou 4, dans lequel ladite fraction est d'environ 62,5 % lorsque l'on définit ladite fenêtre d'une deuxième durée et d'environ 75 % lorsque l'on définit ladite fenêtre d'une première durée.

6. Le stimulateur de la revendication 1, dans lequel les moyens aptes à commander si nécessaire une stimulation auriculaire si la dépolarisation est présente dans la fenêtre de DARA comprennent des moyens aptes, en cas de détection d'une dépolarisation auriculaire à l'intérieur de la fenêtre de DARA, à :
― si aucune autre dépolarisation auriculaire consécutive n'est détectée à l'intérieur de l'IEA déclenché par cette dépolarisation auriculaire : (i) considérer qu'il y a ESA isolée et (ü) lorsque la cavité auriculaire est stimulée à la fin de l'IEA, définir un Délai Atrio-Ventriculaire ou DAV long,
― dans le cas contraire : (i) considérer qu'il y a suspicion de Trouble du Rythme Auriculaire ou TdRA et déclencher une procédure d'analyse du rythme auriculaire et de confirmation/infirmation du TdRA et (ii) définir un DAV court, de durée plus courte que celle du DAV long.

7. Le stimulateur de la revendication 6, dans lequel la durée du DAV long est déterminée en fonction du rythme atrial précédent, le cas échéant compte tenu en outre d'un signal délivré par un capteur d'un paramètre physiologique représentatif du niveau d'activité ou d'effort du patient.

8. Le stimulateur de la revendication 7, dans lequel la durée du DAV long déterminée en fonction du signal délivré par le capteur est plafonnée à une valeur limite prédéterminée.

9. Le stimulateur de la revendication 6, dans lequel la durée du DAV court est une durée fixe liée à la période minimale préprogrammée de stimulation ventriculaire.

10. Le stimulateur de la revendication 1, dans lequel les moyens aptes à commander si nécessaire une stimulation auriculaire si la dépolarisation est présente dans la fenêtre de DARA comprennent des moyens aptes à :
- si une dépolarisation auriculaire est détectée à l'intérieur de la fenêtre de DARA et si au moins une autre dépolarisation auriculaire consécutive est détectée à l'intérieur de l'IEA déclenché par cette dépolarisation auriculaire, considérer alors qu'il y a suspicion de Trouble du Rythme Auriculaire ou TdRA et déclencher une procédure d'analyse du rythme auriculaire et de confirmation/infirmation du TdRA,
- si une proportion majoritaire de x1 % de cycles cardiaques sur M1 cycles successifs vérifient le critère de suspicion de TdRA lors de l'analyse du rythme auriculaire, ou une proportion majoritaire de x2 % de cycles cardiaques sur M2 cycles successifs vérifient le critère de suspicion de TdRA, avec M2 > M1 et x2 < x1, confirmer alors la présence d'un TdRA,
- si la présence d'un TdRA est ainsi confirmée à l'issue de la procédure d'analyse, commuter alors le stimulateur en mode à stimulation ventriculaire désynchronisée.

11. Le stimulateur de la revendication 10, dans lequel M2 = 2.M1, notamment M1 = 32 et M2 = 64, et x1 = 87,5 % et x1 = 56 % environ.

12. Le stimulateur de la revendication 1, dans lequel les moyens aptes à commander si nécessaire une stimulation auriculaire si la dépolarisation est présente dans la fenêtre de DARA comprennent des moyens aptes à :
― si une dépolarisation auriculaire est détectée à l'intérieur de la fenêtre de DARA et si au moins une autre dépolarisation auriculaire consécutive est détectée à l'intérieur de l'IEA déclenché par cette dépolarisation auriculaire, considérer alors qu'il y a suspicion de Trouble du Rythme Auriculaire ou TdRA et déclencher une procédure d'analyse du rythme auriculaire et de confirmation/infirmation du TdRA,
― si le maintien d'un TdRA est confirmé à l'issue de la procédure d'analyse, commuter alors le stimulateur en mode à stimulation ventriculaire désynchronisée et déclencher une nouvelle procédure d'analyse du maintien du TdRA,
― si la disparition du TdRA est détectée lors de cette nouvelle procédure d'analyse du rythme auriculaire, commuter alors le stimulateur en mode de resynchronisation progressive de la stimulation ventriculaire vers une stimulation synchronisée et déclencher une procédure d'analyse des rythmes ventriculaire et auriculaire et de confirmation de la disparition du TdRA,
― si la disparition du TdRA est confirmée lors de cette procédure d'analyse des rythmes ventriculaire et auriculaire, commuter alors le stimulateur en mode synchronisé avec stimulation double-chambre ou mode DDD.

## Patentansprüche

1. Ein Zweikammer-Herzschrittmacher, aufweisend einen Modus der Desynchronisation der ventrikulären Stimulation wenn der Herzvorhofrhythmus ein zulässiges Niveau überschreitet, darauf progressive Resynchronisation im Fall der Rückkehr des Herzvorhofrhythmus auf ein zulässiges Niveau, wobei dieser Schrittmacher umfasst:
a) dazu geeignete Mittel, zu jeder Erkennung einer Herzvorhof-Depolarisation ein Fenster der Erkennung der Beschleunigung des Herzvorhofrhythmus oder DARA zu definieren, dessen Dauer abhängt vom vorderen Herzvorhofrhythmus, bewertet nach der Dauer eines oder mehrerer vorhergehender Herzvorhofintervalle, und
b) dazu geeignete Mittel, bei Erkennen der darauffolgenden Herzvorhof-Depolarisation den Abfluss eines Auslassintervalls des Herzvorhofs oder IEA zu berechnen, und
c) dazu geeignete Mittel, wenn diese Depolarisation im Fenster der DARA anwesend ist, falls nötig eine darauffolgende Herzvorhofstimulation abhängig von der so bestimmten IEA zu steuern,
wobei der Schrittmacher **dadurch gekennzeichnet ist, dass** die zur Festlegung eines Fensters der DARA geeigneten Mittel geeignete Mittel umfassen, die Dauer des Fensters der DARA abhängig vom vorderen Herzvorhofrhythmus anzupassen durch Definieren eines Fensters von einer ersten Dauer oder eines Fensters von einer zweiten Dauer, welche kürzer als besagte erste Dauer ist, wobei die erste Dauer definiert ist durch die ersten Werte des vorderen Herzvorhofrhythmus und die zweite Dauer definiert ist durch die zweiten Werte des vorderen Herzvorhofrhythmus, welche langsamer als besagte erste Werte sind, um eine Unterscheidung zwischen Herzvorhof-Extrasystolen oder ESA und physiologischen Beschleunigungen des Herzvorhofrhythmus zu erlauben.

2. Schrittmacher nach Anspruch 1, wobei die geeigneten Mittel zur Festlegung eines Fensters der DARA geeignete Mittel umfassen zur Erkennung, ob der vordere Herzvorhofrhythmus den besagten ersten Werten des Herzvorhofrhythmus oder den besagten zweiten Werten des Herzvorhofrhythmus angehört, den Wert des vorderen Herzvorhofrhythmus mit einer Schwelle zu vergleichen und besagtes Fenster einer ersten Dauer, falls der Rhythmus die Schwelle überschreitet, und das besagte Fenster einer zweiten Dauer im gegenteiligen Fall festzulegen.

3. Schrittmacher nach Anspruch 1, wobei die Dauer des Fensters der DARA gleich einem Bruchteil des Intervalls ist, das zwei vordere, auf einander folgende physiologische Herzvorhof-Depolarisationen trennt.

4. Schrittmacher nach Anspruch 1, wobei die Dauer des Fenster der DARA gleich einem Bruchteil des Durchschnitts einer vorherbestimmten Anzahl von Intervallen ist, die zwei vordere sukzessive physiologische Herzvorhof-Depolarisationen trennen.

5. Schrittmacher nach Anspruch 3 oder 4, wobei besagter Bruchteil ungefähr 62,5% ist, wenn besagtes Fenster einer zweiten Dauer festgelegt ist, und ungefähr 75%, wenn besagtes Fenster einer ersten Dauer festgelegt ist.

6. Schrittmacher nach Anspruch 1, wobei die Mittel, die dazu geeignet sind bei Vorliegen der Depolarisation im Fenster der DARA falls notwendig eine Herzvorhofstimulation zu steuern, Mittel umfassen, die im Fall der Erkennung einer Herzvorhof-Depolarisation im Inneren des Fensters der DARA dazu geeignet sind:
- falls keine andere folgende Herzvorhof-Depolarisation innerhalb der von dieser Herzvorhof-Depolarisation ausgelösten IEA vorliegt: (i) zu erwägen, ob eine isolierte ESA vorliegt und (ii) wenn die Herzvorhofkavität am Ende der IEA stimuliert wird, eine lange atrio-ventrikuläre Verzögerung oder DAV festzulegen,
- andernfalls: (i) zu erwägen, ob ein Verdacht auf Störung des Herzvorhofrhythmus oder TdRA vorliegt und eine Analyseprozedur des Herzvorhofrhythmus auszulösen und TdRA zu bestätigen/verneinen und (ii) eine kurze DAV von einer kürzeren Dauer als die der langen DAV festzulegen.

7. Schrittmacher nach Anspruch 6, wobei die Dauer der langen DAV abhängig vom vorhergehenden atrialen Rhythmus festgelegt wird, gegebenenfalls unter Berücksichtigung unter anderem eines Sensors eines physiologischen Parameters, der repräsentativ für das Niveau der Aktivität oder der Anstrengung eines Patienten ist.

8. Schrittmacher nach Anspruch 7, wobei die Dauer der langen DAV, festgelegt abhängig von dem vom Sensor abgegebenen Signal, verkürzt wird auf einen vorherbestimmten Grenzwert.

9. Schrittmacher nach Anspruch 6, wobei die Dauer der kurzen DAV eine feste Dauer ist, gebunden an die vorprogrammierte minimale Periode der ventrikulären Stimulation.

10. Schrittmacher nach Anspruch 1, wobei die Mittel, die dazu geeignet sind, wenn die Depolarisation im Fenster der DARA vorhanden ist falls notwendig eine Herzvorhofstimulation zu steuern, Mittel umfassen, die dazu geeignet sind:
- wenn eine Herzvorhof-Depolarisation innerhalb des Fensters der DARA erkannt wird und wenn wenigstens eine andere, darauffolgende Herzvorhof-Depolarisation innerhalb der von dieser Herzvorhof-Depolarisation ausgelösten IEA erkannt wird, dann zu erwägen, ob ein Verdacht auf Störung des Herzvorhofrhythmus oder TdRA vorliegt und eine Analyseprozedur des Herzvorhofrhythmus auszulösen und TdRA zu bestätigen/verneinen,
- wenn ein mehrheitlicher Anteil von x1% der Herzzyklen über M1 aufeinander folgende Zyklen das Kriterium des Verdachts von TdRA anlässlich der Analyse des Herzvorhofrhythmus bestätigen, oder ein mehrheitlicher Anteil von x2% der Herzzyklen über M2 aufeinander folgende Zyklen das Kriterium des Verdachts der TdRA bestätigen, mit M2 > M1 und x2 < x1, dann das Vorhandensein einer TdRA zu bestätigen,
- wenn somit das Vorhandensein der TdRA am Ende der Analyseprozedur bestätigt ist, den Schrittmacher dann in den Modus der desynchronen ventrikulären Stimulation umzuschalten.

11. Schrittmacher nach Anspruch 10, wobei M2 = 2.M1, insbesondere M1 = 32 und M2 = 64, und x1 = 87,5% und x1 = 56% ungefähr.

12. Schrittmacher nach Anspruch 1, wobei die dazu geeigneten Mittel, falls notwendig eine Herzvorhofstimulation zu steuern, wenn die Depolarisation in dem Fenster der DARA vorliegt, Mittel umfasst, die dazu geeignet sind:
- wenn innerhalb des Fensters der DARA eine Herzvorhof-Depolarisation erkannt wird und wenn wenigstens eine andere, folgende Herzvorhof-Depolarisation innerhalb der durch diese Herzvorhof-Depolarisation ausgelösten IEA erkannt wird, dann zu erwägen, ob ein Verdacht auf Störung des Herzvorhofrhythmus oder TdRA vorliegt und eine Analyseprozedur des Herzvorhofrhythmus auszulösen und TdRA zu bestätigen/verneinen,
- wenn nach Ende dieser Analyseprozedur das Andauern einer TdRA bestätigt wird, dann den Schrittmacher in den Modus einer desynchronen ventrikulären Stimulation umzuschalten und eine neue Analyseprozedur über das Andauern der TdRA auszulösen,
- wenn durch diese neue Analyseprozedur des Herzvorhofrhythmus das Verschwinden der TdRA erkannt wird, den Schrittmacher dann in den Modus der progressiven Resynchronisation der ventrikulären Stimulation in Richtung auf eine synchronisierte Stimulation umzuschalten und eine Analyseprozedur der ventrikulären und Herzvorhofrhythmen auszulösen und das Verschwinden der TdRA zu bestätigen,
- wenn durch diese Analyseprozedur der ventrikulären und Herzvorhofrhythmen das Verschwinden der TdRA bestätigt ist, den Schrittmacher dann in den synchronen Modus mit Doppelkammerstimulation oder den Modus DDD umzuschalten.

## Claims

1. A dual-chamber cardiac pacemaker including a mode with desynchronization of the ventricular stimulation when the atrial rhythm exceeds an acceptable level, then a progressive resynchronization in case the atrial rhythm returns to the admissible level, said pacemaker comprising:
a) means for, every time an atrial depolarization is detected, defining a window of Detection of the Acceleration of the Atrial Rhythm or DAAR the duration of which is a function of the prior atrial rhythm, evaluated from the duration of one or several of the preceding atrial intervals,
b) means for, upon detection of the following atrial depolarization, starting the count of a delay for an Atrial Escape Interval or AEI, and
c) means for, if this depolarization is present in the DAAR window, controlling if necessary a subsequent atrial stimulation according to the AEI thus defined,
said pacemaker being **characterized in that** the means for defining a DAAR window includes means for adapting the duration of the DAAR window as a function of the prior atrial rhythm by defining a window having a first duration or a window having a second duration which is shorter than said first duration, the first duration being defined for first levels of prior atrial rhythm and the second duration being defined for second levels of prior atrial rhythm which are slower than the first levels,
whereby permitting an improved discrimination between atrial extrasystoles or AES and physiological accelerations of the atrial rhythm.

2. The pacemaker of claim 1, wherein the means for defining a DAAR window includes, for determining whether the prior atrial rhythm belongs to said first levels of prior atrial rhythm or to said second levels of prior atrial rhythm, means for comparing the value of the prior atrial rhythm to a threshold and to define said window having a first duration when the rhythm exceeds said threshold and said window having a second duration in the opposite case.

3. The pacemaker of claim 1, wherein the duration of the DAAR window is equal to a fraction of the interval separating two successive prior physiological atrial depolarisations.

4. The pacemaker of claim 1, wherein the duration of the DAAR window is equal to a fraction of the average of a predetermined number of intervals separating two successive prior physiological atrial depolarisations.

5. The pacemaker of claim 3 or 4, wherein said fraction is about 62.5% when one defines said window having a second duration, and about 75% when one defines said window having a first duration.

6. The pacemaker of claim 1, wherein the means for controlling if necessary an atrial stimulation if the depolarization is present in the DAAR window includes means for, in case of detection of an atrial depolarization inside the DAAR window:
― if no other atrial consecutive depolarization is detected inside the AEI triggered by this atrial depolarization: (i) considering that there is an isolated AES and (ii) when the atrial cavity is stimulated at the end of the AEI, defining a long Atrio-Ventricular Delay or AVD,
― in the opposite case: (i) considering that there is a suspicion of Trouble of the Atrial Rhythm or ToAR and releasing a procedure of analysis of the atrial rhythm and of confirmation/non-confirmation of the ToAR and (ii) defining a short AVD, of a shorter duration than that of the long AVD.

7. The pacemaker of claim 6, wherein the duration of the long AVD is determined as a function of the prior atrial rhythm, further considering, if any, a signal delivered by a sensor of a representative physiological parameter of the activity or effort level of the patient.

8. The pacemaker of claim 7, wherein the duration of the long AVD as determined as a function of the signal delivered by the sensor is limited to a predetermined limit value.

9. The pacemaker of claim 6, wherein the duration of the short AVD is a fixed duration related to the pre-programmed minimal period of ventricular stimulation.

10. The pacemaker of claim 1, wherein the means for controlling if necessary an atrial stimulation if the depolarization is present in the DAAR window includes means for:
― if an atrial depolarization is detected inside the DAAR window and if at least one other consecutive atrial depolarization is detected inside the AEI triggered by this atrial depolarization, then considering that there is a suspicion of Trouble of the Atrial Rhythm or ToAR and releasing a procedure of analysis of the atrial rhythm and of confirmation/non-confirmation of the ToAR,
― if a majority proportion of x1 % cardiac cycles over M1 successive cycles meet the ToAR suspicion criterion during the analysis of the atrial rhythm, or a majority proportion of x2 % cardiac cycles over M2 successive cycles meet the ToAR suspicion criterion, with M2 > M1 and x2 < x1, confirming the presence of a ToAR,
― if the presence of a ToAR is thus confirmed at the end of the analysis procedure, then switching the pacemaker to a mode with desynchronization of the ventricular stimulation.

11. The pacemaker of claim 10, wherein M2 = 2.M1, in particular M1 = 32 and M2 = 64, and x1 = 87,5 % and x1 = 56 % approximately.

12. The pacemaker of claim 1, wherein the means for controlling if necessary an atrial stimulation if the depolarization is present in the DAAR window includes means for:
― if an atrial depolarization is detected inside the DAAR window and if at least one other consecutive atrial depolarization is detected inside the AEI triggered by this atrial depolarization, then considering that there is a suspicion of Trouble of the Atrial Rhythm or ToAR and releasing a procedure of analysis of the atrial rhythm and of confirmation/non-confirmation of the ToAR,
― if the maintenance of the ToAR is confirmed at the end of the analysis procedure, then switching the pacemaker to a mode with desynchronization of the ventricular stimulation and releasing a further procedure of analysis of the maintenance of the ToAR,
― if the disappearance of the ToAR is detected in the course of this further procedure of analysis of the atrial rhythm, the switching the pacemaker to a mode with progressive resynchronization of the ventricular stimulation towards a synchronized stimulation and releasing a procedure of analysis of the ventricular and atrial rhythms and of confirmation of the disappearance of the ToAR,
― if the disappearance of the ToAR is confirmed in the course of this procedure of analysis of the ventricular and atrial rhythms, then switching the pacemaker to a synchronized mode with dual-chamber stimulation or DDD mode.
